# EUROPEAN PATENT APPLICATION

(11) **EP 4 670 729 A1**
(43) Date of publication of application: **31.12.2025**
(21) Application number: 25185766.0
(22) Date of filing: 27.06.2025
(51) Int. Cl.: A61K 36/23, A61K 31/728, A61K 31/706, A61K 36/481, A61K 38/39, A61P 13/00, A61P 15/02

(54) **PHARMACEUTICAL OR NUTRACEUTICAL COMPOSITION FOR USE IN THE PREVENTION AND/OR THE TREATMENT OF URO-GYNECOLOGICAL DISORDERS**

(30) Priority: 28.06.2024 IT 202400014935
(71) Applicant: Erbozeta S.p.A., 47894 Chiesanuova (SM)
(72) Inventor: PASTORELLI, Chiara, I-47100 FORLI' (IT); ZAVAGLIA, Roberto, 47891 FALCIANO (SM)
(74) Representative: Aseglio-Gianinet, Romina

(57) **Abstract**

The present invention relates to a pharmaceutical or nutraceutical composition, in the form of a unit dosage for oral administration, comprising collagen peptides, astragalus powder, centella asiatica extract, mononucleotides, hyaluronic acid, for use in the prevention and/or treatment of uro-gynaecological disorders selected from: genitourinary syndrome, vulvovaginal atrophy, scleroatrophic lichen, vulvovaginal itching, vulvovaginal dryness.

## Description

### Field of the invention

The present invention relates to a pharmaceutical or nutraceutical composition for the prevention and/or treatment of uro-gynecological disorders, in particular a pharmaceutical or nutraceutical composition, in the form of a dosage unit for oral administration, comprising collagen peptides, astragalus powder, centella asiatica extract, mononucleotides, hyaluronic acid, for the prevention and/or treatment of uro-gynecological disorders.

### Prior art

Urogynecology is a branch of gynecology that focuses on the diagnosis and treatment of problems related to the female urinary and genital system. This specialization mainly deals with urinary incontinence and female genital prolapse, providing solutions to improve the quality of life of patients. Uro-gynecological problems may manifest through a series of symptoms, among the most common are urinary incontinence, a feeling of heaviness at the genital level, urinary and fecal retention, and difficulties in sexual intercourse. Uro-gynecological disorders therefore represent a broad category of conditions, among the main uro-gynecological disorders are: genitourinary syndrome, vulvovaginal atrophy, scleroatrophic lichen, and symptoms such as vulvovaginal itching and vulvovaginal dryness. Among these, genitourinary syndrome, when it appears in association with menopause, is defined as genitourinary syndrome of menopause (GSM). This term encompasses a wide range of clinical manifestations, attributable to estrogen deficiency, that affect the uro-gynecological tract of postmenopausal women. In addition to menopause, other conditions may cause the onset of GSM, including cancer treatment, radiation therapy, chemotherapy, and adjuvant endocrine therapies such as tamoxifen and aromatase inhibitors. Previously known as vulvovaginal atrophy (VVA), GSM is a condition characterized by a variety of symptoms including, by way of example but not limitation, vaginal dryness, burning, itching, irritation, dyspareunia and vaginal discharge. It may also be accompanied by urinary signs and symptoms such as dysuria, urinary incontinence, and frequent urinary tract infections. This disorder also significantly impairs quality of life, sexual health, and the general well-being of affected individuals.

As women's life expectancy continues to increase worldwide, it is expected that in developed countries women will live more than 30 years beyond natural menopause, which typically occurs between the ages of 48 and 52. This prolonged postmenopausal period results in a greater likelihood for women to experience GSM symptoms. In fact, affecting up to 50% of so-called "middle-aged" and elderly women, uro-gynecological symptoms related to menopause are chronic, progressive and unlikely to improve over time, representing a significant health concern for women in the postmenopausal years. Despite its prevalence and impact, GSM remains an underdiagnosed and poorly treated disorder. About 70% of women who experience these symptoms do not consult a doctor, often perceiving these manifestations as a natural aspect of menopause. Moreover, 75% of patients presenting clinical signs of GSM doubt that their healthcare providers have sufficient knowledge of the treatments available to effectively address these problems.

To date, the treatment of GSM requires a multifaceted approach, involving collaboration between the patient and various specialized professionals, including gynecologists, urologists, physiotherapists, and psychologists: in fact, treatment options may include patient education, lifestyle modifications, physical treatments, non-hormonal therapies and, in some cases, hormonal treatments and hormonal receptor modulators. The adoption of a sequential approach, as highlighted by Palacios et al. [Palacios S, Nappi RE, Cancelo MJ, Sánchez S, Simoncini T. Expert opinion on the treatment of vulvovaginal atrophy with ospemifene based on new evidence. Climacteric. 2023 Aug;26(4):388-91], is advantageous for preserving vaginal health, mitigating signs and symptoms, and improving quality of life and sexual health of postmenopausal women. Given the chronic nature of GSM, early and continuous therapeutic intervention is important to slow progression and prevent recurrence of symptoms. However, the challenge of ensuring adherence to treatments, particularly those applied vaginally, which generally show low persistence among patients, requires a sequential and tailored treatment plan. This strategy promotes interchangeability of treatments over time, aligning with the needs and preferences of individual patients.

In the field of non-hormonal therapies, over-the-counter topical vaginal firming products, such as gels and creams, have been suggested. However, it is important to note that these treatments are often not well accepted by patients, in addition to causing serious alterations to the vaginal ecosystem, which may lead to mucosal erosion, increased vaginal discharge and a higher rate of infections.

Another example of therapy involves treatments aimed at restoring the metabolism and functions of connective tissue. Among these, the most important is biostimulation, which activates the anabolic functions of fibroblasts, particularly enhancing the production of collagen, elastin, and type III hyaluronic acid from their precursors. However, few studies support the application of this treatment as a therapeutic strategy and, as far as we know, none of them supports oral administration for vulvovaginal health during GSM. There is therefore a need to explore alternative therapeutic options aimed at amplifying and extending the effectiveness of intravaginal methods.

The scientific article "GALLI VALERIO et al. 'Safety and efficacy of a class II medical device based on highly purified and standardized plant extracts in the management of postmenopausal patients with vulvar and vaginal atrophy: a single-center prospective observational study', MINERVA OBSTETRICS AND GYNECOLOGY, vol. 76, no. 4, 15 February 2024" describes a single-centre, open-label, prospective and observational study conducted on 50 menopausal women affected by GSM in which a gel based on hyaluronate and a mucoadhesive active ingredient enriched with purified alkylamides from Zanthoxylum bungeanum, triterpenes from Centella asiatica and high molecular weight polysaccharides from Tamarindus indica is administered topically intra-vaginally. The results of the study indicate a reduced sensation of pain during sexual intercourse and increased regular sexual activity, as well as a relief of symptoms such as itching and vaginal dryness. However, the study does not provide indications or suggestions regarding the effectiveness of the individual components, or their combination, in oral administration, since presumably the components of the gel, not being absorbed systemically, do not reach the fibroblasts and do not produce biological effects; the systemic action therefore being limited to a temporary barrier effect on symptoms, without acting on tissue trophism or the extracellular matrix.

Patent document KR20210132794 describes an anti-aging composition containing a mixture of collagen and elastin ATAGE-905 and an anti-aging composition with effects such as enhancing cellular activity, inhibiting collagenase expression, inhibiting elastin activity and promoting keratinocyte differentiation through the addition of vitamin, Centella asiatica extract, pollen and substances similar to fish collagen and elastin. The anti-aging composition may maximise the anti-aging function through the synergistic action between the compositions and may be provided in the form of a food product, thus being more practical and sustainable. However, this patent document concerns a formulation containing generic collagen (not bioactive peptides) and functional ingredients for skin well-being. Moreover, this study was conducted on keratinocytes, and not on fibroblasts, i.e., the cells that produce collagen; for this reason, a study that does not demonstrate activity on fibroblast anabolism cannot demonstrate trophic effect on any connective tissue, including vulvovaginal tissue. This study therefore does not concern the genitourinary field, nor is a trophic effect on vaginal tissues demonstrated.

The possibility of having a system for the prevention and/or treatment of uro-gynecological disorders, such as for example: genitourinary syndrome of menopause, vulvovaginal atrophy, scleroatrophic lichen and symptoms such as vulvovaginal itching and vulvovaginal dryness, which is well accepted by patients, does not require the collaboration between the patient and several specialised professionals, is easy to administer and is not a hormonal therapy, is therefore a widely felt need.

The present invention is based on the unexpected finding that the combination of the active components of the composition exerts a systemic effect in oral administration, presumably due to a synergistic action of the components which also induces the production of new extracellular matrix with measurable and prolonged effects.

### Summary of the invention

An object of the present invention is therefore to provide a system for the prevention and/or treatment of uro-gynecological disorders, such as for example: genitourinary syndrome of menopause, vulvovaginal atrophy, scleroatrophic lichen and symptoms such as vulvovaginal itching and vulvovaginal dryness, which is well accepted by patients, does not require the collaboration between the patient and several specialised professionals, is easy to administer and is not a hormonal therapy.

This object is achieved by a pharmaceutical or nutraceutical composition for the prevention and/or treatment of uro-gynecological disorders as outlined in the appended claims, the definitions of which form an integral part of the present description.

### Brief description of the Figures

The invention will be better understood from the following detailed description of preferred embodiments thereof, given by way of example and therefore not limiting, with reference to the attached Figures, in which:

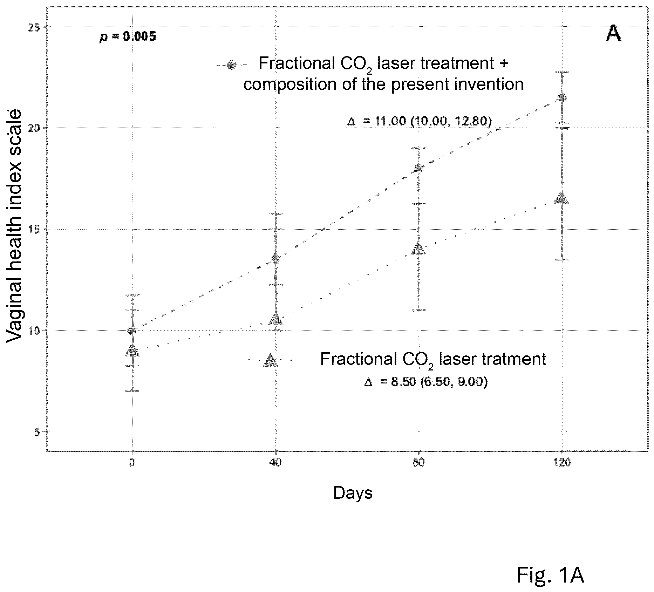

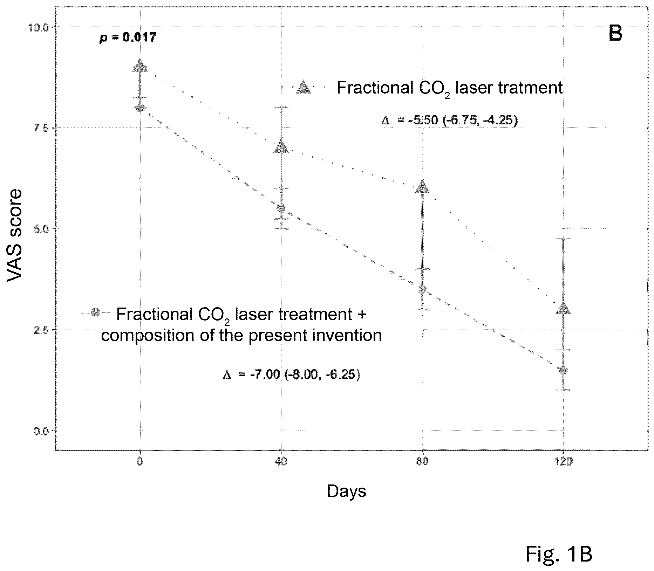

- Figure 1 (1A and 1B) shows two line graphs representing: Figure 1A = median vaginal health index (VHI) (IQR) and Figure 1B = median VAS score (IQR) for pain in patients treated with fractional CO₂ laser treatment alone (MonaLisa Touch^{™}) (Group 1) or with fractional CO₂ laser treatment (MonaLisa Touch^{™}) and concurrent oral administration of the composition according to the present invention at V0 (baseline), V1 (40 days), V2 (80 days), and V3 (120 days);

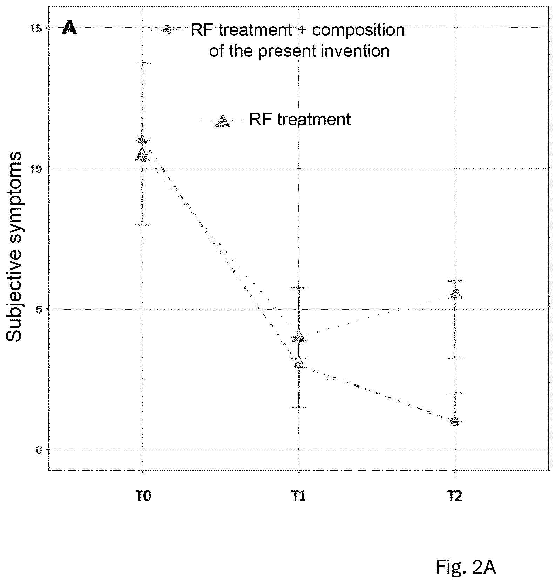

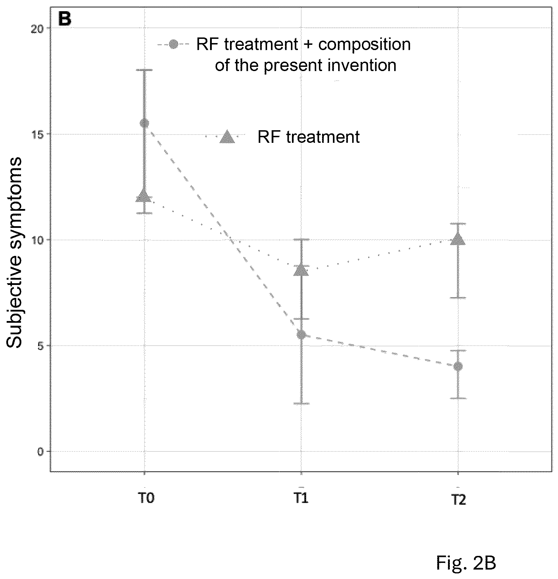

- Figure 2 (2A and 2B) shows two line graphs representing the median total scores (IQR) for subjective symptoms (Figure 2A) and objective signs (Figure 2B) of patients in Group 1 (radiofrequency alone) and Group 2 (radiofrequency and co-administration of the composition according to the present invention) at T0, T1, and T2.

In the attached Figures, identical or similar elements will be denoted by the same reference numerals.

### Detailed description of the invention

An object of the present invention is a pharmaceutical or nutraceutical composition comprising hydrolysed collagen peptides, astragalus powder, centella asiatica extract, RNA mononucleotides, hyaluronic acid having a molecular weight between 3 kDa and 10 kDa, for use in oral administration in the prevention and/or treatment of uro-gynecological disorders selected from: genitourinary syndrome, in particular genitourinary syndrome of menopause (GSM), vulvovaginal atrophy and scleroatrophic lichen.

The term "treatment", in the present invention, includes the therapeutic treatment of the pathologies and/or the alleviation and remission of the typical symptoms of such pathologies, in particular vaginal dryness and vaginal itching.

As known to the skilled person, the term "nutraceutical" means a composition comprising substances derived from plants, food and microbial sources.

With reference to the present description and the attached claims, the term "astragalus" refers to Astragalus, a genus of plants belonging to the Fabaceae (or Leguminosae) family, widespread in the temperate regions of the northern hemisphere. This genus includes over 3000 species.

With reference to the present description and the attached claims, the term "centella asiatica" refers to a medicinal plant belonging to the Apiaceae or Umbelliferae family. Native to the pantropical Asian region, it also grows in Australia and Africa, especially in humid and swampy environments. Today it is particularly widespread in India. Commonly referred to as the "tiger of the grass", it contains a number of pentacyclic triterpenoids generally called centelloids, produced as secondary metabolites. Among these are asiaticoside, madecassoside, and centelloside.

As known to the skilled person, scleroatrophic lichen is a chronic inflammatory disease affecting the skin and mucous membranes, predominantly in the ano-genital area; it is considered a multifactorial inflammatory disease with a probable autoimmune pathogenesis that mostly affects genetically predisposed individuals.

Vulvovaginal itching and vulvovaginal dryness, in addition to being two independent uro-gynecological disorders, may be symptoms related to and coexisting with other mentioned uro-gynecological disorders.

Collagen represents one of the most important structural proteins in the human body. The physiological ageing process of the human body begins around the age of 35, when collagen synthesis starts to undergo both quantitative and qualitative changes. This alteration results in a loss of tone and elasticity of connective tissue, with the consequent appearance of various signs and symptoms depending on the body area affected. These normally slow changes accelerate during menopause due to hormonal reorganisation.

The female genital organs, like all other organs, also undergo physiological changes in shape and volume, going through the various stages of growth: from adolescence, to sexual maturity, to possible pregnancies, and up to menopause.

The lamina propria of the vaginal mucosa, on which the covering epithelium rests, is raised in folds and is composed of dense connective tissue (rich in collagen fibres), elastic fibres, and the vascular-lymphatic network embedded in the extracellular matrix. The most represented cell population in vaginal connective tissue is that of fibroblasts, whose function is to produce and process both the fibrillar component and the extracellular matrix, which consists of glycoproteins, glycosaminoglycans, and proteoglycans. The matrix exerts a deep control on the connective tissue cells since the latter constantly detect and respond to changes in their surrounding environment through interaction with membrane receptors, the integrins, which, once activated, transfer information from the matrix to the cells, influencing their activity, metabolism and physiological cell cycle. After processing the extracellular components, fibroblasts remain quiescent until a catabolic state stimulates them to become metabolically active again. Integrins may also respond to stimulation from various exogenous matrix proteins such as, advantageously, the collagen peptides of the composition according to the present invention which are mistakenly perceived as degraded remnants of collagen produced by the body itself. To balance this false loss, fibroblasts synthesise de novo collagen and extracellular matrix that restore compactness, elasticity and hydration to the vaginal mucosa. Advantageously, therefore, the collagen of the composition according to the present invention is capable of influencing the metabolism of endogenous collagen, increasing the compactness, elasticity and degree of hydration of the tissues of the vaginal mucosa. The collagen of the composition according to the present invention may be of any origin, but is preferably of animal origin (such as, for example, bovine, ovine, porcine or marine collagen) or vegetable origin. Preferably, the collagen of the composition according to the present invention comes from safe and certified sources and is preferably one of the 28 types of collagens identified to date in the prior art. Even more preferably, it is type I, type II, or type III collagen.

Advantageously, the astragalus powder and the centella asiatica extract contained in the composition according to the present invention stimulate the endogenous synthesis of collagen and hyaluronic acid. They also reduce degradation and improve the absorption of proline and glucosamine, amplifying the effectiveness of collagen peptides.

The astragalus powder may be powder of any part of the plant. The centella asiatica extract may be any type of centella asiatica extract with any titration.

Advantageously, the hyaluronic acid contained in the composition according to the present invention contributes to the structural stability of connective tissue and exerts a hydrating action that helps maintain the shape, plasticity, and turgor of the vaginal mucosa. The hyaluronic acid of the composition according to the present invention may be of animal or vegetable origin and may have any molecular weight.

Advantageously, the nucleotides of the composition according to the present invention are nucleotides produced in controlled environments following the most rigorous procedures to guarantee the highest safety standards, have important plastic-reconstructive functions (each new cell requires about 1 billion to form), act as immunomodulators and exert a direct effect on maintaining the integrity of the intestinal and vaginal mucosa.

The composition according to the present invention is therefore, advantageously, a pharmaceutical or nutraceutical composition with trophic action on the vaginal mucosa and urethral epithelium, which stimulates the endogenous production of collagen and elastin. It is indicated in the prevention and treatment of thinning and loss of elasticity of uro-gynecological tissues and in associated sexual disorders, possibly also in combination with pharmacological and physical therapies.

The composition according to the present invention is therefore a composition for use in oral administration in the prevention and/or treatment of uro-gynecological disorders selected from: genitourinary syndrome of menopause, vulvovaginal atrophy, and scleroatrophic lichen, and for the alleviation and remission of typical symptoms of such pathologies such as vulvovaginal itching and vulvovaginal dryness. According to a preferred embodiment of the composition according to the present invention, the uro-gynecological disorder is preferably scleroatrophic lichen. According to an alternative embodiment of the composition according to the present invention, the uro-gynecological disorder is genitourinary syndrome of menopause. According to an alternative embodiment of the composition according to the present invention, the uro-gynecological disorder is preferably vulvovaginal atrophy.

According to a preferred embodiment of the composition according to the present invention, the composition, in dosage unit form, preferably comprises:
- from 1 g to 10 g of said hydrolysed collagen peptides, preferably the hydrolyzation is obtained by enzymatic means, wherein the peptides preferably have a molecular weight between 1.5 kDa and 2.5 kDa, even more preferably have an average molecular weight of 2 kDa,
- from 1 mg to 2 g of astragalus powder,
- from 1 mg to 2 g of centella asiatica extract, of which preferably from 0.005 mg to 10.08 mg of total saponins and from 0.008 mg to 16 mg of asiaticoside,
- from 1 mg to 2 g of RNA mononucleotides,
- from 1 mg to 1 g of hyaluronic acid having a molecular weight between 3 kDa and 10 kDa.

According to a preferred embodiment of the composition according to the present invention, the composition preferably comprises:
- 2.5 g of said hydrolysed collagen peptides,
- 250 mg of astragalus powder and centella asiatica extract,
- 100 mg of RNA mononucleotides,
- 75 mg of hyaluronic acid having a molecular weight between 3 kDa and 10 kDa.

According to a preferred embodiment of the composition according to the present invention, the composition further preferably comprises glucosamine, more preferably from 1 mg to 2 g of glucosamine, even more preferably 500 mg of glucosamine.

As known to the skilled person, glucosamine is an amino-monosaccharide and one of the main precursors of the synthesis of glycosylated proteins and lipids. It is a precursor of hyaluronic acid.

Glucosamine is attributed with anti-inflammatory functions. Furthermore, being a precursor of hyaluronic acid, it plays a proactive role in the prevention of skin blemishes typical of ageing, such as wrinkles and fine lines, contributes to the structural stability of connective tissue and exerts a hydrating action that helps maintain the shape, plasticity and turgor of the vaginal mucosa.

According to a preferred embodiment of the composition according to the present invention, the collagen peptides are preferably hydrolysed collagen peptides obtained by enzymatic means, wherein said peptides preferably have a molecular weight between 1.5 kDa and 2.5 kDa, even more preferably have an average molecular weight of 2 kDa, wherein the mononucleotides are RNA mononucleotides obtained from yeast by biotechnological means.

Advantageously, preclinical studies have shown that, although cellular integrins may interact with a variety of generic collagen peptides, hydrolysed collagen peptides interact optimally with cellular integrins, producing a strong and lasting activation signal in the target cells.

According to a preferred embodiment of the composition according to the present invention:
- the collagen peptides are preferably hydrolysed collagen peptides as indicated above,
- the astragalus powder is preferably astragalus root powder,
- the centella asiatica extract is preferably centella asiatica leaf extract,
- the mononucleotides are preferably selected from: 5'-AMP, free acid of adenosine
5'-monophosphate; 5'-CMP, free acid of cytidine 5'-monophosphate; 5'-UMP, disodium salt of uridine 5'-monophosphate; 5'-GMP, disodium salt of guanosine 5'-monophosphate; 5' -IMP, disodium salt of inosine 5'-monophosphate, and mixtures of two or more of these,
- the hyaluronic acid is preferably hyaluronic acid having a molecular weight between 3 kDa and 10 kDa.

Preferably, the composition according to the present invention comprises a mixture of five of said eutrophic nucleotides derived from enzymatic hydrolysis of yeast RNA.

Advantageously, hyaluronic acid, being preferably hyaluronic acid with very low molecular weight (3-10 kDa), plays an important biological role by exerting different effects on cellular behaviour and a different penetration into tissues. A study conducted on the ability of hyaluronic acid to cross the intestinal epithelium showed that only hyaluronic acid with a molecular weight equal to or less than 5 kDa can more easily cross the intestinal barrier and that absorption is proportional to its concentration.

The composition according to the present invention may be administered at any time of the day (morning, afternoon or evening). Preferably, the use of said composition involves the administration of from 2.9 g to 11.7 g of the composition according to the present invention. Even more preferably from 2.9 g to 8.7 g. Even more preferably from 2.9 g to 5.8 g. Even more preferably it is 2.9 g.

Preferably, the administration is 2.9 g of the composition according to the present invention, at any time of the day, for 4 consecutive months. The administration may be repeated after 4 months, for a total of 2 treatment cycles per year.

Preferably, the composition according to the present invention is for oral administration in a human subject who is concurrently undergoing, or has undergone, fractional carbon dioxide (CO₂) laser treatment or radiofrequency pulse treatment for the treatment of genitourinary syndrome of menopause.

As known to the skilled person, carbon dioxide (CO₂) laser treatment is a fractional ablative treatment, which promotes selective photothermolysis targeting water molecules within cells. Whereas radiofrequency pulse treatment is a non-invasive method using a specific radiofrequency system. This high-energy system deeply heats the tissues by means of highfrequency electromagnetic waves, thereby stimulating collagen and increasing tissue turgor, improving circulation and restoring adequate blood supply to ensure better hydration and transpiration of the vaginal mucosa.

Preferably, the fractional carbon dioxide (CO₂) laser treatment is conducted for about 20 minutes, preferably using pulses having a power between 20 W and 50 W, interspersed with rest times between 300 µs and 1500 µs.

Preferably, the radiofrequency pulse treatment is conducted for about 15-30 minutes, preferably using pulses having a power between 20 W and 35 W, interspersed with rest times of about 500 ms.

As described above, each component of the composition according to the present invention has been shown to exert the following effects:
- the hydrolysed collagen peptides influence the metabolism of endogenous collagen, thereby increasing the compactness, elasticity and degree of hydration of uro-gynecological tissues;
- the astragalus powder and centella asiatica extract stimulate the endogenous synthesis of both collagen and hyaluronic acid;
- the hyaluronic acid contributes to the structural stability of connective tissue and exerts a hydrating action that helps maintain the shape, plasticity and turgor of the uro-gynecological mucosa;
- the mononucleotides have a plastic-reconstructive function useful for the maintenance of the integrity of the uro-gynecological mucosa.

The composition according to the present invention, being a composition comprising all the above components, is advantageously capable of exerting all the above effects. Surprisingly, moreover, the characteristic of comprising in a single composition collagen peptides, astragalus powder, centella asiatica extract, mononucleotides and hyaluronic acid further provides the advantage of generating a synergistic effect among all the components of the composition: the astragalus powder and the centella asiatica extract, by stimulating both endogenous collagen synthesis and endogenous hyaluronic acid synthesis, amplify the effectiveness of both the collagen peptides and the hyaluronic acid of the composition according to the present invention, thereby further contributing to amplify the effectiveness of the nucleotides of the composition according to the present invention as maintainers of the integrity of the uro-gynecological mucosa.

The composition according to the present invention therefore has the advantages of being a pharmaceutical or nutraceutical composition for the prevention and/or treatment of uro-gynecological disorders, such as for example: genitourinary syndrome of menopause, vulvovaginal atrophy, scleroatrophic lichen, and symptoms such as vulvovaginal itching and vulvovaginal dryness, which is well accepted by patients, whose administration and hence preventive and/or therapeutic effect does not require the collaboration between the patient and several specialised professionals, is easy to administer and is not a hormonal therapy.

When the composition according to the present invention further comprises glucosamine, the composition advantageously shows a greater preventive and/or therapeutic effect against uro-gynecological disorders, such as for example: genitourinary syndrome of menopause, vulvovaginal atrophy, scleroatrophic lichen, and symptoms such as vulvovaginal itching and vulvovaginal dryness. This greater preventive and/or therapeutic effect is mainly due to the fact that glucosamine is a precursor of hyaluronic acid. Glucosamine is indeed attributed with anti-inflammatory functions. It is a substance that, being a precursor of hyaluronic acid, contributes to the structural stability of connective tissue and exerts a hydrating action that helps maintain the shape, plasticity and turgor of the vaginal mucosa.

When the composition according to the present invention is for oral administration in a human subject who is concurrently undergoing, or has undergone, fractional carbon dioxide (CO₂) laser treatment or radiofrequency pulse treatment for the treatment of genitourinary syndrome of menopause, the composition according to the present invention advantageously shows a further greater preventive and/or therapeutic effect against genitourinary syndrome of menopause. This greater preventive and/or therapeutic effect is mainly due to the synergistic effect that has been shown to occur when the composition according to the present invention is administered in a human subject who is concurrently undergoing, or has undergone, fractional carbon dioxide (CO₂) laser treatment or radiofrequency pulse treatment.

The preferred embodiments of the present invention, given by way of example and not limitation, set out below, are therefore provided as examples of the preventive and/or therapeutic effects of the composition according to the present invention when administered in a human subject who is concurrently undergoing, or has undergone, fractional carbon dioxide (CO₂) laser treatment or radiofrequency pulse treatment.

### Examples

### EXAMPLE 1: Preventive and/or therapeutic effect of the composition according to the present invention when administered orally in a human subject who is concurrently undergoing, or has undergone, to fractional carbon dioxide (CO₂) laser treatment for the treatment of genitourinary syndrome of menopause.

### Study design and population

A cohort study was conducted on 20 postmenopausal volunteer women over the age of 50 diagnosed with GSM. All patients underwent cervical-vaginal cytological screening performed within the year and had a negative urine culture. Women suffering from vulvovaginal infections, bladder infections or presenting abnormal uterine bleeding were excluded from the study. The patients were randomly divided into two groups: Group 1, composed of 10 patients, underwent three sessions of fractional carbon dioxide (CO₂) laser treatment (MonnaLisa Touch^{™}), spaced 40 days apart. Group 2, also composed of 10 patients, underwent the same three sessions of fractional carbon dioxide (CO₂) laser treatment (MonnaLisa Touch^{™}), spaced 40 days apart, and in combination with the laser treatment, they were administered daily (for 120 days) the composition according to the present invention containing 2.5 g of collagen peptides, 75 mg of hyaluronic acid, 250 mg of astragalus powder and centella asiatica extract, and 100 mg of mononucleotides.

### Outcome measurements and study timing

For all patients, the following data were prospectively collected in an electronic database: age (years), body mass index (BMI; kg/m²), education level, marital status, pregnancies, age at menopause and any comorbidities, including a history of previous surgical procedures. Patients were assessed at four key time points:
- V1, is the beginning of the study and corresponds to the first session of fractional CO₂ laser treatment for both groups and the start of oral administration of the composition according to the present invention exclusively for Group 2;
- V2 and V3, scheduled respectively at the second and third laser treatment sessions;
- V4, corresponding to 40 days after the last laser treatment session and the end of oral administration of the composition according to the present invention for Group 2.

The evaluation considered the signs of GSM, the perception of pain related to vaginal atrophy, and sexual function. The signs of GSM were assessed using the vaginal health index (VHI) before each session of fractional CO₂ laser treatment (V1, V2 and V3) and 40 days after the last one (V4). The VHI assessed each of the following characteristics on a scale from 1 (worst scenario) to 5 (best scenario): vaginal elasticity, secretions, pH level, epithelial integrity and hydration levels. Total VHI scores ranged from 5 to 25, with a cut-off of <15 adopted to indicate an atrophic vagina. The perception of pain related to GSM was assessed using the Visual Analogue Scale (VAS) (0-10) before each session of fractional CO₂ laser treatment (V1, V2 and V3) and 40 days after the last one (V4). Sexual function was assessed using the Female Sexual Function Index (FSFI) questionnaire before the first session of fractional CO₂ laser treatment (V1) and 40 days after the last one (V4).

As a secondary measure, patient adherence, in both groups, was assessed using a satisfaction survey administered at the end of the study (V4), where scores ranged from 0 (completely dissatisfied, no perceived benefit) to 20 (completely satisfied). In addition, any adverse events reported during the study were documented.

### Treatments

The MonnaLisa Touch^{™} fractional CO₂ laser treatment (DEKA, Florence, Italy) is a fractional ablative intravaginal treatment delivered every 30-45 days for three consecutive months. Each treatment session lasts about 20 minutes. The settings of the fractional micro-ablative CO₂ laser used in this study for treatment of the vaginal canal were as follows: D-Pulse mode, dot power 40 W; dwell time 1000 µs and spot spacing 1000 µm. For the treatment of the vaginal introitus, the settings were: dot power 24 W; dwell time 400 µs; spot spacing 1000 µm. The procedure was performed on an outpatient basis and did not require any specific preparation or anaesthesia. The patients were placed in the dorsal lithotomy position. Vaginal sexual intercourse was advised against for at least three days after laser application to prevent an inflammatory reaction that could last up to 48 hours. Only Group 2 patients concurrently received oral administration of the composition according to the present invention. The composition was administered once daily, starting from V1 for a duration of 120 days.

### Baseline characteristics of the patients

The characteristics of the patients are detailed in Table 1. Overall, the median age was 54 (IQR: 52 - 59) years and the median BMI (body mass index) was 23.5 (IQR: 22.1 - 25.7) kg/m². There were no significant differences in demographic and clinical characteristics between the different treatment groups.

### Outcome measurements: vaginal health index

At baseline (V1), the median VHI was 9.00 for Group 1 and 10.00 for Group 2 (p=0.4). During treatment, the VHI for Group 1 increased to 10.50, 14.00, and 16.50 at V2, V3, and V4 respectively; the improvements for Group 2 were more pronounced, with scores increasing to 13.50, 18.00, and 21.50 at V2, V3, and V4 respectively (Table 2). As shown in Figure 1A, the difference in VHI values between the final evaluation (V4) and the initial one (V1) was greater for women who received the combined treatment (Group 2) compared to those who received only fractional CO₂ laser treatment (Group 1): Δ=11.00 vs. Δ=8.50, p=0.005. Women in Group 2 reached a VHI >15 (cut-off for vaginal atrophy) earlier than those in Group 1 (V3 vs. V4).

### Outcome measurements: pain assessment

The median VAS at baseline (V1) for pain was 9.00 in patients in Group 1 and 8.00 in those in Group 2 (p=0.3). During the study, these scores decreased to 7.00, 6.00 and 3.00 at V2, V3, V4 respectively in women in Group 1, and to 5.50, 3.50 and 1.50 at V2, V3 and V4 respectively in women in Group 2 (Table 2). As shown in Figure 1B, the difference in VAS values between the final evaluation (V4) and the initial one (V1) was greater for women who received the combined treatment (Group 2) compared to those who received only fractional CO₂ laser treatment (Group 1): Δ=-7.00 vs. Δ=-5.50, p=0.017.

### Outcome measurements: sexual function

The median FSFI for Group 1 improved from 51.50 at baseline (V1) to 60.50 at the end of the study and, for Group 2, from 53.50 at baseline (V1) to 67.50 at the end of the study (Table 1).

### Outcome measurements: satisfaction and safety profile

Treatment satisfaction levels were also higher in Group 2, with a median score of 18.00 (IQR: 18.00 - 18.00) compared to 11.50 (IQR: 8.75 - 18.00) in Group 1 (p=0.047). Group 2 also reported high scores for each domain evaluated regarding oral administration of the composition according to the present invention: 9.00 (IQR: 8.25 - 9.75) for taste, 9.00 (IQR: 9.00 - 9.75) for administration method, 10.00 (IQR: 9.00 - 10.00) for gastrointestinal tolerability, and 9 (IQR: 9.00 - 9.75) for duration of therapy. No side effects were reported in either group.

**Table 1**

| **Characteristics** | **Total N=20 (100%)** | **Group 1 N=10 (50%)** | **Group 2 N=10 (50%)** | **p-value²** |
|---|---|---|---|---|
| **Age (years)** | 54 (52; 59) | 55 (48; 60) | 54 (52; 57) | 0.9 |
| **BMI (kg/m²)** | 23.5 (22.1; 25.7) | 23.1 (21; 24.2) | 25.2 (22.7; 25.8) | 0.3 |
| **Education level** | | | | 0.9 |
| Middle school diploma | 2 (10.0%) | 1 (10.0%) | 1 (10.0%) | |
| High school diploma | 6 (30.0%) | 3 (30.0%) | 3 (30.0%) | |
| University degree | 12 (60.0%) | 6 (60.0%) | 6 (60.0%) | |
| **Number of pregnancies** | 1 (1; 2) | 2 (0; 2) | 1 (1; 2) | 0.9 |
| **Duration of menopause (years)** | 5 (3; 9) | 7 (5; 10) | 5 (3; 8) | 0.4 |
| **Marital status** | | | | 0.4 |
| Single | 6 (30.0%) | 4 (40.0%) | 2 (20.0%) | |
| Married | 12 (60.0%) | 6 (60.0%) | 6 (60.0%) | |
| Separated/Divorced | 10 (10.0%) | 0 (0%) | 2 (20.0%) | |
| **Previous surgical interventions** | 5 (25.0%) | 2 (20.0%) | 3 (30.0%) | 0.9 |

Table 1: descriptive characteristics of the study population.

Group 1: fractional CO₂ laser treatment only (MonaLisa Touch^{™}). Group 2: fractional CO₂ laser treatment (MonaLisa Touch^{™}) and composition according to the present invention. Abbreviations: BMI = body mass index.

¹Median (IQR); n(%); ²Wilcoxon rank-sum test; Exact Wilcoxon rank-sum test; Fisher's exact test.

**Table 2**

| | **Group 1** | | | |
|---|---|---|---|---|
| | **Fractional CO₂ laser treatment only (MonaLisa Touch^{™})** | | | |
| | V1 | V2 | V3 | V4 |
| VHI | 9,00 (7.00 - 11.00) | 10.50 (10.00 - 15.75) | 14.00 (11.00 - 19.00) | 16.5 (13.5 - 20.0) |
| VAS | 9.00 (8.25 - 9.00) | 7.00 (5.25 - 8.00) | 6.00 (4.00 - 6.00) | 3.00 (2.00 - 4.75) |
| FSFI | 51.50 (41.25 - 63.75) | - | - | 60.50 (48.25 - 72.00) |

| | **Group 2** | | | |
|---|---|---|---|---|
| | **Fractional CO₂ laser treatment (MonaLisa Touch^{™}) and composition according to the present invention** | | | |
| | V1 | V2 | V3 | V4 |
| VHI | 10.00 (8.25 - 11.75) | 13.50 (12.25 - 15.00) | 18.00 (16.25 - 19.00) | 21.50 (20.20 - 22.80) |
| VAS | 8.00 (8.00 - 9.00) | 5.50 (5.00 - 6.00) | 3.50 (3.00 - 4.00) | 1.00 (1.00 - 2.00) |
| FSFI | 53.50 (46.50 - 59.75) | - | - | 67.50 (61.00 - 75.00) |

Table 2: Scores of subjective symptoms and objective signs at each specified time point in patients treated only with fractional CO₂ laser treatment (MonaLisa Touch^{™}) (Group 1) and with fractional CO₂ laser treatment (MonaLisa Touch^{™}) and the composition according to the present invention (Group 2).

### Discussion of the results

The results of the present study showed: i) an objective improvement in vaginal health assessed through the VHI, ii) a self-reported reduction in pain related to GSM assessed by the VAS scale, and iii) an increase in sexual function assessed through the FSFI questionnaire in both patient groups. However, the magnitude of such changes was consistently greater in women in Group 2 who received the combined protocol compared to those who received only fractional CO₂ laser treatment, with statistically significant differences in median values even 40 days after the last session of fractional CO₂ laser treatment. Furthermore, patients who underwent the combined protocol (Group 2) reported higher levels of satisfaction with their treatment.

The comparison of data from Group 1 with those from Group 2 is also indicative of the activity of the composition according to the invention even in the absence of laser treatment.

### EXAMPLE 2: Preventive and/or therapeutic effect of the composition according to the present invention when administered orally in a human subject who is concurrently undergoing, or has undergone, to radiofrequency pulse treatment for the treatment of genitourinary syndrome of menopause.

### Study population

The study was conducted on 20 postmenopausal volunteer women suffering from GSM. All patients, who signed informed consent for study enrolment, data collection and analysis, underwent cervical-vaginal cytological screening performed within the year and had a negative urine culture. Women suffering from vulvovaginal infections, bladder infections or presenting abnormal uterine bleeding were excluded from the study. Patients were randomly assigned (1: 1) into two treatment groups: 10 patients (Group 1) underwent vulvovaginal radiofrequency (RF) pulse treatment only and 10 patients (Group 2) underwent vulvovaginal RF pulse treatment in combination with oral administration of the composition according to the present invention.

### Radiofrequency treatment

The transcutaneous temperature-controlled RF (TTCRF) device used in the present study is a bipolar RF device (SECTUM, Nrose, Neauvia) at 480 kHz. The treatment programme consisted of four treatments, with each session spaced two weeks apart. A generous amount of hyaluronic acid in gel form was applied to the disposable handpiece, serving both as a moisturiser and conductor. The handpiece was inserted into the vagina and the RF treatment was carried out with an energy setting of 25-30 W in continuous mode, with a total duration of 15 minutes from the moment the average internal vaginal temperature reached approximately 38°C, never exceeding 40°C. The current was delivered in pulsed mode (500 ms), for 15-20 minutes, with slow movements along the entire vaginal canal. Subsequently, with the application of a generous amount of hyaluronic acid gel to the vulva, the treatment continued using the same probe also in pulsed mode, performing slow movements for another 10 minutes.

### Treatment with administration of the composition according to the present invention

Only the patients in Group 2 concurrently received a systemic treatment, with oral administration of 2.5 g of the composition according to the present invention. The treatment was administered once daily for four months.

### Assessment of parameters and study timing

For all patients, the following data were prospectively recorded in an electronic database: age (years), body mass index (BMI; kg/m²), education level, marital status, pregnancies, age at menopause, and any comorbidities, including history of previous surgical interventions. To study the effects of the treatments on GSM, a checklist was used to systematically assess five subjective symptoms (vaginal dryness, dyspareunia, irritation/burning/itching, dysuria and bleeding during sexual activity) and six objective signs (elasticity, vaginal rugae, fluid secretion, epithelial thickness, moisture, and tissue colour), along with vaginal pH. These assessments were conducted at three time points: T0, marking the start of RF treatment for both Groups, with Group 2 also beginning oral supplementation simultaneously; T1, two months later, at the end of RF treatment; and T2, five months from T0, providing a posttreatment observation window. The initial phase between T0 and T1 focused on a direct comparison between the two Groups to observe the immediate effects of treatment. Each sign and symptom were rated on a four-level scale (0 = none/normal, 1 = mild, 2 = moderate, 3 = severe), allowing detailed comparison of treatment outcomes. Subsequently, the period extending to T2 served as an observation phase to monitor the lasting impacts and long-term benefits of oral supplementation to RF treatment. Vaginal pH was measured using test strips (Vaginal Health series, Just Fitter), at the beginning of treatment and at the same time points mentioned above. Accordingly, each measurement was assigned a score as follows: >6.1 = -1; 5.6 to 6.0 = -2; 5.1 to 5.5 = -3; 4.6 to 5 = -4; and <4.5 = -5. Adverse events, compliance, and product tolerability were also assessed during the study.

### Results

Baseline demographic and clinical characteristics of the patient population stratified by treatment received (Group 1: vulvovaginal RF alone vs. Group 2: vulvovaginal RF and concurrent oral administration of the composition according to the present invention) are shown in Table 3. The total score for subjective symptoms and objective signs assessed at each specified time point, as well as the score assigned to each specific domain, are shown in Table 4 and Figure 2.

Initially, at T0, the median total score for subjective symptoms was 10.50 (IQR: 8.00; 11.00) vs. 11.0 (IQR: 10.25; 13.75) in patients in Group 1 and Group 2, respectively (p = 0.2). The median total score for objective signs was 12.00 (IQR: 11.25; 18.00) compared to 15.50 (IQR: 12.00; 18.00) in patients in Group 1 and Group 2, respectively (p = 0.4). The median score for vaginal pH was 1.5 (IQR: 1.0; 2.0) in both Groups (p = 0.9).

At the end of RF treatment (T1), the median total score for subjective symptoms dropped to 4.00 (IQR: 3.25; 5.75) in Group 1 patients and to 3.00 (IQR: 1.50; 4.00) in Group 2 patients (p = 0.2). The median total score for objective signs decreased to 8.50 (IQR: 6.25; 10.00) in Group 1 patients and to 5.50 (IQR: 2.25; 8.75) in Group 2 patients (p = 0.2). Median scores for vaginal pH increased to 4.5 (IQR: 4.0; 5.0) and 5.0 (IQR: 4.0; 5.0) respectively in patients in Group 1 and Group 2 (p = 0.6).

Three months after the last RF treatment session (T2), the median total score for subjective symptoms slightly increased in Group 1 patients (5.50; IQR: 3.25; 6.00), while it continued to decrease in Group 2 patients (1.00; IQR: 1.00; 2.00) (p < 0.001). The same occurred for objective signs: after the interruption of RF treatment in Group 1 patients, the median total score increased to 10.00 (IQR: 7.25; 10.75), while it continued to decrease to 4.00 (IQR: 2.50; 4.75) in women in Group 2 (p < 0.001), who, after discontinuing RF treatment, continued with oral administration of the composition according to the present invention for another 2 months, advantageously demonstrating that the composition alone, when administered orally, is capable of preventing and/or treating GSM. Finally, the median vaginal pH score at T2 was 4.0 (IQR: 3.0; 4.0) and 4.5 (IQR: 4.0; 5.0) in patients in Group 1 and Group 2, respectively (p = 0.015).

No side effects were reported from the oral administration of the composition according to the present invention.

**Table 3**

| **Characteristics** | **Group 1 N=10 (50%)¹** | **Group 2 N=10 (50%)¹** | **p-value²** |
|---|---|---|---|
| **Age (years)** | 56 (53; 58) | 56 (52; 58) | 0.7 |
| **BMI (kg/m²)** | 25.1 (24.6; 25.7) | 25.6 (23.5; 27.8) | 0.6 |
| **Education level** | | | 0.7 |
| Middle school diploma | 2 (20.0%) | 4 (40.0%) | |
| High school diploma | 5 (50.0%) | 4 (40.0%) | |
| University degree | 3 (30.0%) | 2 (20.0%) | |
| **Number of pregnancies** | 2 (1; 3) | 2 (1; 3) | 0.5 |
| **Age at menopause (years)** (anni) | 51 (50; 51) | 50 (50; 51) | 0.8 |
| **Marital status** | | | 0.9 |
| Single | 1 (10.0%) | 2 (20.0%) | |
| Married | 7 (70.0%) | 7 (70.0%) | |
| Separated/Divorced | 2 (20.0%) | 1 (10.0%) | |

Table 3: Descriptive characteristics of the study population.

Group 1: vaginal treatment with radiofrequency pulses only. Group 2: vaginal treatment with RF pulses in combination with oral administration of the composition according to the present invention. Abbreviations: BMI = body mass index.

¹Median (IQR); n(%); ²Wilcoxon rank-sum test; Fisher's exact test.

**Table 4**

| | **Group 1 Vulvovaginal RF** | | |
|---|---|---|---|
| | T0 | T1 | T2 |
| *Subjective symptoms* | | | |
| Vaginal dryness | 3.00 (2.00 - 3.00) | 2.00 (2.00 - 2.00) | 2.00 (1.00 -2.00) |
| Dyspareunia | 3.00 (2.00 - 3.00) | 1.00 (1.00 - 1.75) | 2.00 (1.25 - 2.00) |
| Irritation/burning/itching | 2.00 (2.00 - | 1.00 (1.00 | 1.00 (1.00 |
| | 2.00) | - 1.00) | - 1.00) |
| Dysuria | 1.50 (1.00 - 2.00) | 0 (0 - 1.00) | 1.00 (0 - 1.00) |
| Bleeding with sexual activity | 1.00 (1.00 - 1.00) | 0 (0 - 0) | 0 (0 - 0) |
| TOTAL | 10.50 (8.00 - 11.00) | 4.00 (3.25 - 5.75) | 5.50 (3.25 - 6.00) |

| *Objective signs* | | | |
|---|---|---|---|
| Elasticity | 2.00 (2.00 - 3.00) | 1.50 (1.00 - 2.00) | 2.00 (1.25 - 2.00) |
| Vaginal rugae | 2.00 (2.00 - 3.00) | 2.00 (1.00 - 2.00) | 2.00 (1.00 - 2.00) |
| Fluid secretion | 2.00 (2.00 - 3.00) | 1.00 (1.00 - 1.00) | 1.00 (1.00 - 1.75) |
| Epithelial thickness | 2.00 (1.25 - 3.00) | 1.50 (1.00 - 2.00) | 2.00 (2.00 - 2.00) |
| Moisture | 2.00 (2.00 - 3.00) | 1.00 (1.00 - 1.00) | 1.00 (1.00 - 1.00) |
| Tissue colour | 2.00 (2.00 - 3.00) | 1.50 (1.00 - 2.00) | 2.00 (1.00 - 2.00) |
| TOTAL | 12.00 (11.25 - 18.00) | 8.50 (6.25 - 10.00) | 10.00 (7.25 - 10.75) |

| | **Group 2 Vulvovaginal RF + composition according to the present invention** | | |
|---|---|---|---|
| *Subjective symptoms* | | | |
| Vaginal dryness | 3.00 (2.25 - 3.00) | 1.00 (1.00 - 2.00) | 1.00 (0.25 - 1.00) |
| Dyspareunia | 3.00 (2.00 - 3.00) | 1.00 (0.25 - 1.00) | 0 (0 - 1.00) |
| Irritation/burning/itching | 2.00 (2.00 - 2.75) | 0 (0 - 1.00) | 0 (0 - 0) |
| Dysuria | 2.00 (2.00 - 3.00) | 0 (0 - 1.00) | 0 (0 - 0) |
| Bleeding with sexual activity | 1.00 (1.00 - 2.00) | 0 (0 - 0) | 0 (0 - 0) |
| TOTAL | 11.00 (10.25 - 13.75) | 3.00 (1.50 - 4.00) | 1.00 (1.00 - 2.00) |

| *Objective signs* | | | |
|---|---|---|---|
| Elasticity | 3.00 (2.00 - 3.00) | 1.00 (0 - 2.00) | 1.00 (1.00 - 1.75) |
| Vaginal rugae | 2.50 (2.00 - 3.00) | 1.00 (1.00 - 2.00) | 1.00 (1.00 - 1.00) |
| Fluid secretion | 2.50 (2.00 - 3.00) | 1.00 (0 - 1.00) | 0 (0 - 0) |
| Epithelial thickness | 2.50 (2.00 - 3.00) | 1.00 (1.00 - 1.75) | 1.0 (1.0 - 1.0) |
| Moisture | 2.50 (2.00 - 3.00) | 1.00 (0 - 1.00) | 0 (0 - 0) |
| Tissue colour | 2.50 (2.00 - 3.00) | 1.00 (0 - 1.75) | 1.00 (0.25 - 1.00) |
| TOTAL | 15.50 (12.00 - 18.00) | 5.50 (2.25 - 8.75) | 4.00 (2.50 - 4.75) |

Table 4: Scores of subjective symptoms and objective signs, at each time point, in patients treated with vaginal radiofrequency only (Group 1) and vaginal radiofrequency + oral administration of the composition according to the present invention (Group 2).

### Discussion of results

The four sessions of vulvovaginal RF treatment, spaced two weeks apart over a two-month period, both alone (Group 1) and in co-administration with the composition according to the present invention (Group 2), demonstrated improvements in the symptoms and signs of GSM and restoration of vaginal pH. However, the combination therapy (Group 2) advantageously led to significantly better results than RF treatment alone, despite the absence of statistical differences between the two groups at the end of the RF treatment period (T1). Nevertheless, in the follow-up period (T2), while Group 1 showed a recurrence of symptom and sign scores, Group 2 showed further improvement. This divergence was statistically significant at T2, highlighting the enhanced and lasting benefits of both the combination therapy and the sole administration of the composition according to the present invention.

The comparison of data from Group 1 with those from Group 2 is also to be considered indicative of the activity of the composition according to the invention even in the absence of treatment with radiofrequency pulses.

## Claims

1. Pharmaceutical or nutraceutical composition, comprising hydrolysed collagen peptides, astragalus powder, centella asiatica extract, RNA mononucleotides, hyaluronic acid having a molecular weight comprised between 3 kDa and 10 kDa, for use in oral administration in the prevention and/or treatment of uro-gynecological disorders selected from: genitourinary syndrome of menopause, vulvovaginal atrophy, scleroatrophic lichen.

2. Composition for use according to claim 1, wherein the uro-gynecological disorder is scleroatrophic lichen.

3. Composition for use according to claim 1 or 2 in the form of unit dosage, comprising:
- 1 g to 10 g of the aforementioned hydrolysed collagen peptides,
- 1 mg to 2 g astragalus powder,
- 1 mg to 2 g centella asiatica extract, of which 0.005 mg to 10.08 mg total saponins and 0.008 mg to 16 mg asiaticoside.
- 1 mg to 2 g RNA mononucleotides,
- from 1 mg to 1 g of hyaluronic acid having a molecular weight comprised between 3 kDa and 10 kDa.

4. Composition for use according to any one of claims 1 to 3, comprising:
- 2.5 g of said hydrolysed collagen peptides,
- 250 mg astragalus powder and centella asiatica extract,
- 100 mg RNA mononucleotides,
- 75 mg hyaluronic acid having a molecular weight comprised between 3 kDa and 10 kDa.

5. Composition for use according to any one of claims 1 to 4, further comprising glucosamine, preferably 1 mg to 2 g glucosamine, even more preferably 500 mg glucosamine.

6. Composition for use according to any one of claims 1 to 5, wherein the collagen peptides are hydrolysed collagen peptides obtained by enzymatic means, wherein said peptides have a molecular weight comprised between 1.5 kDa and 2.5 kDa, and wherein the mononucleotides are RNA mononucleotides obtained from yeast by biotechnological means.

7. Composition for use according to any one of claims 1 to 6, wherein:
- the astragalus powder is astragalus root powder,
- the centella asiatica extract is centella asiatica leaf extract,
- the mononucleotides are selected from: 5'-AMP, free acid of adenosine 5'-monophosphate; 5'-CMP, free acid of cytidine 5'-monophosphate; 5'-UMP, disodium salt of uridine 5'-monophosphate; 5'-GMP, disodium salt of guanosine 5'-monophosphate; 5'-IMP, disodium salt of inosine 5'-monophosphate, and mixtures of two or more thereof.

8. Composition for use according to any one of claims 1 to 7, in administration at any time of the day (morning, afternoon or evening).

9. Composition for use according to any one of claims 1 to 8, in oral administration in a human subject who is concurrently undergoing, or has undergone, to fractional carbon dioxide (CO₂) laser treatment or radiofrequency pulse treatment for the treatment of genitourinary syndrome of menopause.

10. Composition for use according to claim 9, wherein the use of said composition comprises administering 2.9 g to 11.7 g of the composition.

11. Composition for use according to claim 9 or 10, wherein the fractional carbon dioxide (CO₂) laser treatment is conducted, preferably for about 20 minutes, using pulses having power comprised between 20 W and 50 W, interspersed with rest times comprised between 300 µs and 1500 µs.

12. Composition for use according to claim 9 or 10, wherein the radiofrequency pulses treatment is conducted, preferably for about 15-30 minutes, using pulses having a power comprised between 20 W and 35 W, interspersed with rest times of about 500 ms.
